Europäisches Patentamt

European Patent Office

Office européen des brevets

(11) Veröffentlichungsnummer: **0 255 041 B1**

## EUROPÄISCHE PATENTSCHRIFT

(45) Veröffentlichungstag der Patentschrift: **24.04.91**

(51) Int. Cl.5: **A61L 2/18**, G02C 13/00,
//(A61L2/18,A01N59:00)

(21) Anmeldenummer: **87110664.7**

(22) Anmeldetag: **23.07.87**

(54) **Desinfektions- und Reinigungsmittelsystem für Kontaktlinsen.**

(30) Priorität: **31.07.86 DE 3626082**

(43) Veröffentlichungstag der Anmeldung:
**03.02.88 Patentblatt 88/05**

(45) Bekanntmachung des Hinweises auf die
Patenterteilung:
**24.04.91 Patentblatt 91/17**

(84) Benannte Vertragsstaaten:
**AT BE CH DE ES FR GB IT LI NL**

(56) Entgegenhaltungen:
**US-A- 3 873 696**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Wisotzki, Klaus-Dieter, Dr.
Wahnenmühle 4
W-4006 Erkrath(DE)**
Erfinder: **Bansemir, Klaus, Dr.
Ursulaweg 51
W-4018 Langenfeld(DE)**
Erfinder: **Jacobs, Jochen, Dr.
Am Acker 20
W-5600 Wuppertal 1(DE)**
Erfinder: **Kruse, Hans
Am Hallenbad 44
W-4052 Korschenbroich 2(DE)**

**Beschreibung**

Die zunehmende Zahl an Trägern von weichen oder harten Kontaktlinsen macht die Bereitstellung eines in jedem Falle bequem und sicher anwendbaren Systems von Sterilisierungs- und Reinigungsmitteln erforderlich. Hierzu werden vorwiegend Peroxoverbindungen benutzt, die Keime und Pilze zerstören, deren Überschüsse jedoch selbst wieder zerstört werden müssen, bevor die Linsen wieder auf die Pupille gesetzt werden.

So wird in der deutschen Offenlegungsschrift 28 35 652 zum Desinfizieren und Reinigen von Kontaktlinsen ein Redoxsystem beispielsweise aus Ascorbinsäure und in wäßriger Lösung stark alkalisch reagierendem Alkalipercarbonat verwendet, in dessen wäßrige Lösung die Kontaktlinsen etwa 5 Minuten lang hineingelegt werden. Nach dabei erfolgter Desinfektion werden die Linsen gegebenenfalls mit Kochsalzlösung gespült. Dann können sie wieder auf die Augen aufgebracht werden.

Nach der deutschen Offenlegungsschrift 33 29 922 werden Kontaktlinsen dadurch desinfiziert und gereinigt, daß man sie 10 bis 20 Minuten lang in einer Lösung aus Kochsalz und einer darin gelösten Tablette (A) aus Harnstoffperoxohydrat lagert, sie anschließend 15 Minuten lang in einer frischen Lösung aus Kochsalz und einer darin gelösten Tablette (B) aus Natriumascorbat oder einer Mischung aus Ascorbinsäure und Natriumcarbonat beläßt und schließlich noch mindestens 5 Minuten lang in einer reinen Kochsalzlösung lagert. Danach können die Linsen wieder getragen werden.

In der europäischen Patentanmeldung 82 798 wird ein Verfahren zum Desinfizieren und Reinigen von Kontaktlinsen beschrieben, wonach diese zunächst etwa 20 Minuten lang in eine Wasserstoffperoxidlösung gelegt werden, der anschließend zur Zersetzung von $H_2O_2$-Überschüssen eine Katalasetablette zugefügt wird. Das Enzym wirkt innerhalb von 5 Minuten.

Als am bequemsten für den Anwender hat sich bisher offenbar ein Desinfektions- und Reinigungsmittelsystem erwiesen, bei dem beispielsweise Harnstoffperoxohydrat in einer Kochsalzlösung zusammen mit einem kationischen, nichtionischen oder vorzugsweise amphoteren bzw. anionischen Tensid und einem Katalysator für die nachträgliche Zerstörung des Peroxidüberschusses angewendet wird. Ein solches System ist in der amerikanischen Patentschrift 4 414 127 beschrieben. Die gesondert verpackten Systembestandteile, nämlich zum einen das Harnstoffperoxohydrat und zum anderen eine Lösung aus dem Tensid, dem Katalysator und dem Kochsalz, werden unmittelbar vor dem Gebrauch zusammengegeben. Danach braucht sich der Anwender um nichts mehr zu kümmern. Nachteilig ist hier, daß das Tensid an sich zwar beliebiger Natur sein kann, daß aber bevorzugt zwei solche aus sehr speziellen anionischen oder kationischen Tensidgruppen eingesetzt werden sollen. Außerdem wird als Katalysator für die Zersetzung überschüssigen Harnstoffperoxohydrats ein Schwermetallsalz wie zum Beispiel Kupfersulfat verwendet. Die Verwendung von Schwermetallsalzen ist umstritten und regional unterschiedlich gesetzlich reglementiert (vgl. die verschiedenen Abwassergesetze der einzelnen Bundesländer). Die Behandlung der Linsen nimmt allerdings trotzdem bis zu 4 Stunden in Anspruch.

Es wurde nun gefunden, daß man zu ebenfalls gleichzeitig und bequem anwendbaren Desinfektions- und Reinigungsmittelsystemen für harte und weiche Kontaktlinsen mit einem Gehalt an festen Peroxoverbindungen, Reduktionsmitteln und/oder Katalysatoren und Tensiden kommt, wenn der peroxoverbindungshaltige Anteil einen Zusatz an einem Alkylglucosid, und der $H_2O_2$-neutralisierende Anteil Puffersalze zur Einstellung von pH 7 aufweist.

Überraschenderweise wird die desinfizierende Wirkung von Wasserstoffperoxid bzw. $H_2O_2$-liefernden Peroxoverbindungen wie z.B. Kaliumpersulfat und vorzugsweise Harnstoffperoxohydrat, die in wäßriger Lösung sauer reagieren, durch Zugabe von Alkylglucosiden deutlich verbessert.

Als Alkylglucoside kommen solche der allgemeinen Summenformel $R_1O(Z)_x$ in Betracht, wobei $R_1$ eine Alkylkette mit 8-18, vorzugsweise 12 - 14 C-Atomen, Z Glucose und x 1 bis 10, vorzugsweise 1 bis 5 bedeuten .

Diese Alkylglucoside zeichnen sich zusätzlich durch ihre ausgezeichnete Schleimhautverträglichkeit am Auge sowie durch ihre oberflächenaktiven Eigenschaften aus. Sie können vorzugsweise auch mit weiteren verträglichen Tensiden kombiniert werden, wobei mit "verträglichen Tensiden" solche nichtionische Tenside gemeint sind, die die leistungssteigernden antimikrobiellen Eigenschaften nicht rückgängig machen, z. B. Fettalkoholethoxylate, Addukte von Ethylenoxid und Propylenoxid an Fettalkohole oder n-alkyl-endgruppenverschlossene Fettalkoholethoxylate. Mit diesen Tensiden werden in der Hauptsache der Trübungspunkt der Reinigungs- und Desinfektionslösung gesteuert sowie gegebenenfalls deren schaumdämpfende Eigenschaften ausgenutzt.

Bevorzugt werden dem peroxoverbindungshaltigen Anteil des Desinfektions-und Reinigungsmittelsystems noch Hilfsstoffe zugesetzt, vor allem solche, die den pH-Wert während der Reinigung auf pH 2 bis 7, vorzugsweise pH 3,5 einstellen, wie insbesondere Zitronensäure, Salicylsäure oder Milchsäure oder Gemische davon. Diese Hilfsstoffe können die antimikrobielle Aktivität der Lösung zusätzlich deutlich steigern.

Die Alkylglucoside werden mit den pulverförmigen Peroxoverbindungen mechanisch und in bekannter Weise gemischt, wobei das Verhältnis der mittleren Molmassen von Peroxoverbindung zu Alkylglucosid $1 . 10^2 : 1$ bis $32 . 10^4 : 1$, vorzugsweise $1 . 10^3 : 1$ bis $32 . 10^3 : 1$ und insbesondere $1 . 10^3 : 1$ bis $8,3 . 10^3 : 1$ beträgt.

Die beim Desinfektions- und Reinigungsvorgang nicht verbrauchten $H_2O_2$-Mengen werden durch zeitlich leicht verzögerte oder auch gleichzeitige Zugabe von Reduktionsmitteln und/oder Katalysatoren neutralisiert. Geeignete Reduktionsmittel sind z. B. Ascorbinsäure, Natriumascorbat oder Glucose. Als Katalysatoren werden Enzyme eingesetzt. Ein besonders geeignetes und bevorzugtes Enzym ist Katalase. Die Aktivität der Katalase wird in Units/mg angegeben. Eine Sigma-Unit baut, ausgehend von einer Konzentration von 10,3 μmol/ml in der Reaktionsmischung, bei pH 7 und 25 °C in 1 Minute 1 μmol $H_2O_2$ ab. Die Bestimmung erfolgt über die Messung der Absorption bei 240 nm.

Wenn man nur mit Reduktionsmittel arbeitet, benötigt man davon mindestens äquimolare Mengen in bezug auf die Peroxoverbindung. Dabei kann sich allerdings bei der Neutralisation zuviel Wärme entwickeln, die unter Umständen linsenschädigend wirken kann. Arbeitet man nur mit Katalysatoren, d. h. Enzymen, so geht die Neutralisation des restlichen $H_2O_2$ zwar schnell aber nur bei Raumtemperatur vor sich und die Vorteile einer Wärmeentwicklung entfallen. Deshalb hat sich die Kombination eines Reduktionsmittels mit Katalase als günstig für die Neutralisation erwiesen, weil die Reinigungslösung dann bei einsetzender Neutralisation schwach erwärmt wird und Wärme dabei den zeitlichen Ablauf und die Reinigungswirkung z. B. in bezug auf die Fettentfernung günstig unterstützt.

Die Menge an Reduktionsmittel entspricht bei alleinigem Einsatz der der eingesetzten Peroxoverbindung, d. h. es werden äquimolare Mengen mit einem Überschuß von 1 bis 5, vorzugsweise 2 bis 4 Mol-% an Reduktionsmittel eingesetzt. Bei gemeinsamem Einsatz mit einem Enzym kann die Menge des Reduktionsmittels auf etwa die Hälfte bis auf etwa ein Viertel reduziert werden.

Das Enzym wird je nach Aktivität in Mengen von 0,001 bis 0,2 mg/ml Lösung, vorzugsweise von 0,005 bis 0,1 mg/ml Lösung eingesetzt, bezogen auf die Menge der Peroxoverbindung.

Neben dem Reduktionsmittel und dem Enzym enthält das Neutralisationsmittel für die Reduktion der $H_2O_2$-Reste des Desinfektions-und Reinigungsmittelsystems gleichzeitig noch Puffersalze, die den pH-Wert der Gesamtlösung auf ca. pH 7 einstellen, wie Natriumhydrogencarbonat, Natriumcarbonat oder Natriumcitrat. Es kann nach Bedarf zusätzlich noch die schon angesprochenen verträglichen Tenside und/oder zusätzliche Salze wie z. B. Natriumchlorid und/oder Farbstoffe enthalten.

Die Bestandteile des erfindungsgemäßen Desinfektions- und Reinigungsmittelsystems können insgesamt jeweils in wäßriger Lösung, als Pulver, aber auch als Tablette vorliegen. Bevorzugte Angebotsformen sind die Tablettenformen. Es muß dafür Sorge getragen werden, daß es während des Lagerzeitraums nicht zur direkten Berührung zwischen der Peroxoverbindung mit dem Tensid und dem Neutralisationsmittelgemisch kommt. Die Verpackung muß jeweils luft- und vor allem feuchtigkeitsdicht verschlossen sein, um ein vorzeitiges Zersetzen der Peroxoverbindung zu vermeiden.

Wenn das erfindungsgemäße System in Tablettenform angeboten wird, so wird der Zusatz weiterer Tablettierhilfsstoffe notwendig. Für eine rasche Auflösung können insbesondere Sprengmittel zweckmäßig sein. Beispielhaft genannt seien Celluloseether oder Derivate, Polyethylenglycole, Polyvinylalkohole und Polyvinylpyrrolidon.

Um bei gleichzeitigem Zusammengeben sämtlicher Bestandteile eine leichte Verzögerung der Löslichkeit des Neutralisationsmittelgemisches zu bewirken, wird dieses zweckmäßigerweise mit wasserlöslichen filmbildenden Polymeren besprüht, die die Mischungsbestandteile weitgehend überziehen und sich zuerst selbst auflösen müssen, bevor das Wasser die reagierenden Substanzen erreicht. Auf diese Weise "versiegelte" Mischungen können auch zu Tabletten verpreßt werden.

Die bevorzugte Angebotsform ist die in Tabletten. Man kann dann praktisch mit gleichzeitiger Befüllung des Kontaktlinsenreinigungsbehälters arbeiten, indem man z. B. zwei Einzeltabletten, die sich mit Verzögerung in Wasser oder Kochsalzlösung auflösen, einsetzt. Aber auch zwei verschiedene Pulver oder gemischte Angebotsformen von Pulvern und Tabletten garantieren dem Kontaktlinsenträger eine hohe Anwendungssicherheit. Mögliche Verwechslungen können zusätzlich durch unterschiedliches Anfärben der desinfizierenden und der neutralisierenden Bestandteile vermieden werden. Bei Einsatz der vorteilhaften Angebotsformen erfolgt nach der Desinfektion und Reinigung sofort anschließend die Reduktion, ohne daß der Kontaktlinsenträger gesondert tätig zu werden braucht.

Die Desinfektion und Reinigung kann bei Temperaturen von 10 bis 60 °C, vorzugsweise von ca. 35 °C im Wasserglas oder einer anderen geeigneten Vorrichtung, durchgeführt werden. Diese Temperaturen können sich einerseits bei einsetzender Reduktion selbst entwickeln und einen günstigen Einfluß auf die Reinigungsleistung nehmen, andererseits können sowohl Desinfektion, Reinigung als auch Reduktion schon bei erhöhter Temperatur stattfinden, wenn die Lösung z. B. durch eine Heizquelle erwärmt wird. In diesem Falle könnte der Gehalt an Reduktionsmittel im Neutralisationsgemisch weiter verringert werden, so daß dann im wesentlichen das Enzym allein den Abbau des überschüssigen $H_2O_2$ bewirkt.

Es sei noch bemerkt, daß unter "Neutralisieren" im erfindungsgemäßen Sinne die Zerstörung von überschüssigem $H_2O_2$ verstanden werden soll. Die hierfür verwendeten Mittel sind als "Neutralisationsmittel" bezeichnet worden.

Versuchsteil

Die folgenden Beispiele mit den darin aufgeführten Untersuchungsergebnissen zeigen die Vorteile des erfindungsgemäßen Desinfektions- und Reinigungsmittelsystems für Kontaktlinsen gegenüber dem Stand der Technik in Analogie zur amerikanischen Patentschrift 4 414 127.

I.) Desinfektionswirkung

Die Desinfektionswirkung wurde im quantitativen Suspensionstest nach den zeitlich modifizierten Richtlinien der deutschen Gesellschaft für Hygiene und Mikrobiologie (Zbl. Bakt. Hyg., 1. Abt. Orig. B 172 (1981) 534) mit folgenden Keimen durchgeführt:
1. Staphylococcus aureus
2. Pseudomonas aeruginosa
3. Candida albicans
4. Aspergillus niger

Dabei wurden einer Keimsuspension mit ca. $10^8$ Keimen/ml 0,1 ml entnommen und zu 10 ml der erfindungsgemäßen Desinfektions-und Reinigungslösung gegeben, wodurch die Prüfkonzentration bei etwa $10^6$ Keimen/ml lag. Von Desinfektion wird dann gesprochen, wenn eine Keimreduktion um $10^5$ Keime bzw. 5 log-Stufen erfolgt. Prüfkonzentration und Wahl der Testkeime entsprechen auch den Bestimmungen der British Pharmacopoeia 1980, Addendum 1982, Appendix XVI.

II.) Reinigungswirkung

Zur Überprüfung der Reinigungswirkung wurden Kontaktlinsen unterschiedlicher Materialzusammensetzung künstlich angeschmutzt; folgende drei Anschmutzungen wurden hergestellt:
a) Fettanschmutzung
b) Proteinanschmutzung
c) anorganische Anschmutzung

a) Fettanschmutzung

Die Fettanschmutzung setzte sich aus folgenden Bestandteilen zusammen (pro 100 g Lösung):
0,010 g Tripalmitin
0,100 g Triolein
0,100 g Trilinolein
0,005 g Cholesterylpalmitat
0,035 g Cholesteryloleat
0,050 g Cholesteryllinoleat

Die Lipide wurden eingewogen und zuerst miteinander verschmolzen, bevor mit destilliertem $H_2O$ auf 100 g aufgefüllt wurde. Von der Lösung wurden je 3 ml genommen, um je eine Kontaktlinse zu überschichten. Die Lösung wurde dann innerhalb von ca. 16 Stunden bei 35 °C bis fast zur Trockene eingeengt, wobei sich die Lipide als nicht abspülbare Anschmutzung auf der Kontaktlinsenoberfläche ablagerten.

b) Proteinanschmutzung

0,04 g Mucin
0,18 g Albumin
0,08 g alpha-Globulin
0,08 g beta-Globulin
0,08 g gamma-Globulin
0,18 g Lysozym

Die Proteine wurden zuerst in Kochsalzlösung vorgelöst und diese Lösung dann mit destilliertem Wasser auf 100 g aufgefüllt. Die Ablagerung auf einer Kontaktlinse erfolgte wie unter a) beschrieben, zusätzlich wurde die Ablagerung noch 30 Minuten lang mit UV-Licht bestrahlt.

c) anorganische Anschmutzung

Die Herstellung der anorganischen Anschmutzung geschah folgendermaßen:
Lösung 1 1,25 g $Na_2HPO_4$, 0,975 g $NaH_2PO_4$, 1,09 g $NaHCO_3$ wurden in 100 g destilliertem Wasser gelöst
Lösung 2 0,065 g $CaCl_2$ wurden ebenfalls in 100 g destilliertem Wasser gelöst

Die Kontaktlinsen wurden angeschmutzt, indem man eine Kontaktlinse in eine Mischung aus 2,9 ml der Lösung 1 und 0,1 ml der Lösung 2 eintauchte und wie unter a) beschrieben eine nichtabspülbare Anschmutzung erzeugte.

Das für die Untersuchungen eingesetzte Leitungswasser hatte folgende Qualität:

$Ca^{2+}$ 2,12 mol/m³
$Mg^{2+}$ 0,46 mol/m³
$Na^+$ 3,44 mol/m³
$K^+$ 0,12 mol/m³
$Zn^{2+}$ 0,3 mmol/m³

$Fe^{2+}$ 0,9 mmol/m³
$Cu^{2+}$ 0,0 mmol/m³
$Mn^{2+}$ 0,4 mmol/m³
Si 0,14 mol/m³
$Cl^-$ 3,78 mol/m³
$SO_4^{2-}$ 0,67 mol/m³
pH-Wert 7,4
Leitfähigkeit 92 mS/m

Beispiel 1

1,5 g Harnstoffperoxohydrat, 1 mg eines $C_{12-14}$-Alkylglucosids mit x = 1,4, 2 mg eines nichtionischen Tensids bestehend aus dem mit 1 Mol Butylalkohol verethertem Umsetzungsprodukt aus 1 Mol eines $C_{12-14}$-Fettalkoholgemisches und 9 Mol Ethylenoxid und 0,13 g Zitronensäure wurden in 10 ml destilliertem Wasser gelöst. In der Lösung stellte sich bei einem pH-Wert von 3,5 eine Wasserstoffperoxidkonzentration von 5 Gew.-% ein.

Im quantitativen Suspensionstest mit Staphylococcus aureus wurde bereits nach 2 Minuten eine Keimreduktion um 6 log-Stufen erreicht. Gleiche Ergebnisse wurden auch in Abwesenheit des zusätzlichen nichtionischen Tensids erreicht. Die vollständig tensidfreie Lösung erreichte diese antimikrobielle Leistung erst nach 7,5 Minuten. Die Zugabe von dem konventionellen nichtionischen Tensid allein führte zu keiner Verringerung dieser Zeit. Das Beispiel zeigt die Verbesserung der keimtötenden Wirkung der Lösung nach Zugabe sehr geringer Mengen Alkylglucosid.

Beispiel 2

Ersetzte man in Beispiel 1 das Alkylglucosid durch ein nach der amerikanischen Patentschrift 4 414 127 eingesetztes Tensid, nämlich Miranol C2M konz. ᴿ der Firma Miranol Chemical Corp., so wurde im quantitativen Suspensionstest am gleichen Testkeim eine Keimreduktion um 6 log-Stufen erst nach 4 Minuten erreicht.

Gegenüber der tensidfreien Lösung konnte damit zwar eine nach dem Stand der Technik auch ausgelobte Leistungssteigerung beobachtet werden, gemäß Beispiel 1 ist die Leistungssteigerung erfindungsgemäß jedoch deutlich stärker ausgeprägt.

Als weiterer Vorteil gegenüber dem Stand der Technik ist die erfindungsgemäß äußerst geringe Tensidkonzentration zu erwähnen. In der amerikanischen Patentschrift 4 414 127 werden weitaus höhere Tensidkonzentrationen, nämlich ab 0,1 Gew.-%, bevorzugt aber 1 % beansprucht.

Beispiel 3

Ersetzte man in Beispiel 1 das Alkylglucosid durch ein Oxoalkoholethoxylat (z. B. Isotridecanol × 9 Mol Ethylenoxid = Marlipal 013/90 ᴿ) der Firma Hüls A.G., so wird bei gleichen Versuchsbedingungen eine Keimreduktion um 6 log-Stufen auch erst nach 4 Minuten erreicht.

Beispiel 4

Ersetzte man in Beispiel 1 das Alkylglucosid durch ein Kondensationsprodukt aus Ethylenoxid und Propylenoxid (Pluronic L 61 ᴿ) der Firma Wyandotte, so wurde bei gleichen Versuchsbedingungen eine Keimreduktion um 6 log-Stufen erst nach 5 Minuten erreicht.

Die Beispiele 1 - 4 zeigen damit die Vorteile der erfindungsgemäßen Tenside gegenüber denen des Standes der Technik und gegenüber anderen nichtionischen Tensiden.

Beispiel 5

Zusammen mit der Lösung gemäß Beispiel 1 wurden zusätzlich 0,03 g Salicylsäure aufgelöst. Die Geschwindigkeit der Desinfektion wurde nach dem quantitativen Suspensionstest nochmals gesteigert. Am Testkeim Staphylococcus aureus konnte die Desinfektionszeit zur Erreichung der gewünschten Keimreduktion um 6 log-Stufen noch um 1 Minute verkürzt werden.

Candida albicans wurde mit einer Lösung aus Beispiel 1 nach einer Einwirkzeit von 5 Minuten desaktiviert (5 log-Stufen). Die gleiche Desinfektionsleistung wurde durch Zusatz von Salicylsäure bereits nach 3 Minuten erreicht.

Pseudomonas aeruginosa wurde von einer Lösung gemäß Beispiel 1 bereits nach 30 Sekunden desaktiviert (6 log-Stufen), eine Leistungssteigerung gemäß Beispiel 5 konnte meßtechnisch nicht mehr erfaßt werden.

Beispiel 6

Der quantitative Suspensionstest wurde auch bei erhöhter Temperatur (35 °C) durchgeführt.

Die Ergebnisse mit den Ansätzen nach den Beispielen 1 und 5 zeigen, daß eine Temperaturerhöhung auf 35 °C eine weitere Verkürzung der Desinfektionszeit zur Folge hat.

Beispiel 7

Kontaktlinsen wurden mit einem Keimgemisch, bestehend aus je 2 Tropfen der Keime Staphylococcus aureus, E. Coli, Pseudomonas aeruginosa und Candida albicans (Keimzahl $7 \times 10^8$), 5 Minuten lang kontaminiert und 10 Minuten lang auf sterilem Filterpapier getrocknet. Danach wurden die Linsen bei Raumtemperatur in einer Petrischale mit 10 ml Produkt gemäß Beispiel 1 bzw. Beispiel 5 desinfiziert. Die Kontaktlinsen konnten bei Raumtemperatur innerhalb von 5 Minuten vollständig dekontaminiert werden.

Beispiel 8

In Analogie zu Beispiel 7 erfolgte 5 Minuten lang eine Kontaminierung der Kontaktlinsen mit Aspergillus niger in einer Keimsuspension bestehend aus $3 \times 10^7$ Keimen.

Mit einer Lösung gemäß Beispiel 5 waren die Kontaktlinsen bei Raumtemperatur ebenfalls nach 5 Minuten desinfiziert.

Beispiel 9

Kontaktlinsen wurden wie beschrieben mit Ablagerungen a), b) und c) angeschmutzt und mit einer Lösung gemäß Beispiel 1 bzw. Beispiel 5 gereinigt.

Die Kontrolle der Reinigungswirkung erfolgte mit einem Stereomikroskop SV 8 der Firma Zeiss.

Bei der Fettentfernung konnte durch das Alkylglucosid innerhalb von ca. 5 Minuten eine Reinigung erzielt werden. Die analoge tensidfreie Lösung zeigte eine deutlich geringere Wirkung.

Die Proteinentfernung erfolgte ebenfalls nach ca. 5 Minuten. Dadurch erübrigte sich die sonst notwendige wöchentliche Intensivreinigung.

Anorganische Beläge waren durch den schwach sauren Charakter der Lösungen innerhalb von 2 Minuten vollständig von den Linsen entfernt.

Beispiel 10

Analog zu Beispiel 9 wurde die Reinigung der Kontaktlinsen bei 35 °C durchgeführt. Insbesondere bei der Fettentfernung konnten die Vorteile einer kürzeren Reinigungszeit beobachtet werden.

Beispiel 11

Eine Tablette, bestehend aus 1,5 g Harnstoffperoxohydrat, 1 mg $C_{8-10}$-Alkylglucosid mit $x = 1,8$, 2 mg nichtionisches Tensid (gemäß Beispiel 1), 0,13 g Zitronensäure, 0,03 g Salicylsäure und 0,05 g Natriumchlorid, wurde zusammen mit 10 ml 0,9%iger Natriumchloridlösung und den zu reinigenden Kontaktlinsen in einen Reinigungsbehälter gegeben.

Die Tablette löste sich innerhalb von ca. 2 Minuten auf. Desinfektion und Reinigung waren nach 5 Minuten abgeschlossen. In den Behälter wurde danach eine weitere Tablette gegeben, bestehend aus 0,6 mg Katalase (10000 - 25000 Units/mg nach Sigma), 0,38 g Natriumascorbat, 0,25 g Natriumhydrogencarbonat, 33 mg Polyethylenglykol mit MG 6000 (Polywachs 6000 $^R$) und 2 mg eines Kondensationsproduktes aus einem $C_{16-18}$-Fettalkohol und 20 Mol Ethylenoxid (Eumulgin B 2 $^R$).

Diese zweite Tablette löste sich in ca. 3 Minuten, neutralisierte dabei überschüssige Peroxidmengen und stellte gleichzeitig einen pH-Wert um ca. 7 ein. Die gereinigten Kontaktlinsen wurden dann dem Behälter entnommen, unter fließendem Leitungswasser abgespült und waren danach wieder weitgehend augenverträglich.

In einer Versuchsvariante wurde anstelle des Abspülvorgangs mit fließendem Leitungswasser ein kurzes Eintauchen der behandelten Kontaktlinsen in isotonische Kochsalzlösung vorgenommen, bevor diese dann von den Trägern wieder eingesetzt wurden. Über Unverträglichkeiten wurde nicht geklagt.

Beispiel 12

In Weiterführung von Beispiel 11 wurden zusätzlich zu den schon beschriebenen Bestandteilen der 1. Tablette 0,01 g EDTA-Dinatriumsalz in diese eingearbeitet.

Diese Tablette wurde zusammen mit 10 ml Leitungswasser und den zu reinigenden Kontaktlinsen in einen Reinigungsbehälter gegeben. Nach dem Auflösen der Tablette erfolgte die Desinfektion und Reinigung der Kontaktlinsen innerhalb von ca. 5 Minuten. Die Neutralisation erfolgte wie in Beispiel 11 beschrieben.

Beispiel 13

Ein Pulvergemisch, bestehend aus 1,5 g Harnstoffperoxohydrat, 1 mg Alkylglucosid (gemäß Beispiel 1), 2 mg nichtionisches Tensid (gemäß Beispiel 1), 0,13 g Zitronensäure und 0,03 g Salicylsäure, wurde aus einer gas- und feuchtigkeitsdichten Tüte zusammen mit den zu reinigenden Kontaktlinsen in einen Reinigungsbehälter mit 10 ml dest. Wasser gegeben. Das Pulver löste sich sehr schnell auf. Desinfektion und Reinigung waren innerhalb von ca. 5 Minuten abgeschlossen. Die Neutralisation erfolgte wie in Beispiel 11 beschrieben.

Beispiel 14

Eine Tablette, bestehend aus 1,5 g Harnstoffperoxohydrat, 1 mg Alkylglucosid, 2 mg Mischether, 0,05 g Natriumchlorid, 0,13 g Zitronensäure und 0,03 g Salicylsäure, wurde gleichzeitig mit einer zweiten Tablette, bestehend aus einem Gemisch aus 0,36 g Natriumascorbat, 0,6 mg Katalase (10000 - 25000 Units/mg nach Sigma), 0,24 g Natriumhydrogencarbonat und 0,032 g Polywachs 6000 $^R$, auf das vor dem Verpressen 9 mg eines neutralisierten Polyacrylates eingesprüht wurden, zusammen mit den angeschmutzten Kontaktlinsen und 10 ml isotonischer Kochsalzlösung in einen Behälter gegeben. Die erste Tablette löste sich dabei innerhalb von ca. 3 Minuten und lieferte die für die Desinfektion und Reinigung erforderliche Lösung.

Die zweite Tablette löste sich aufgrund ihres Gehaltes an Polyacrylat, das die einzelnen Mischungsbestandteile teilweise umhüllte, mit leichter zeitlicher Verzögerung innerhalb von ca. 12 Minuten, reduzierte dabei überschüssige Mengen Peroxid und stellte einen pH-Wert von 7 ein.

Die Reinigungslösung wurde ausgegossen und die Kontaktlinsen nach Eintauchen in eine isotonische Kochsalzlösung wieder auf die Augen gebracht. Sie verursachten keine Beschwerden.

Beispiel 15

Entsprechend der Zusammensetzung wie in Beispiel 14 wurden die Bestandteile der ersten Tablette hier in Pulverform vorgelegt und mit der in Beispiel 14 beschriebenen zweiten Tablette kombiniert. Die Ergebnisse deckten sich erwartungsgemäß mit denen von Beispiel 14.

**Ansprüche**

1. Desinfektions- und Reinigungsmittelsystem für Kontaktlinsen mit einem Gehalt an festen Peroxoverbindungen, Reduktionsmitteln und/oder Katalysatoren und Tensiden, dadurch gekennzeichnet, daß der peroxoverbindungshaltige Anteil einen Zusatz an einem Alkylglucosid und der $H_2O_2$-neutralisierende Anteil Puffersalze zur Einstellung von pH 7 aufweist.

2. System nach Anspruch 1, dadurch gekennzeichnet, daß der peroxoverbindungshaltige Anteil aus Kaliumpersulfat oder Harnstoffperoxohydrat besteht.

3. System nach Anspruch 1 und 2, dadurch gekennzeichnet, daß das Alkylglucosid der allgemeinen Summenformel $R_1O\ (Z)_x$ entspricht, wobei $R_1$ eine Alkylkette mit 8 - 18, vorzugsweise 12 - 14 C-Atomen, Z Glucose und x 1 bis 10, vorzugsweise 1 bis 5 bedeuten.

4. System nach Anspruch 1 bis 3, dadurch gekennzeichnet, daß der Anteil aus Peroxoverbindung und Alkylglucosid einen Zusatz an einem damit verträglichen nichtionischen Tensid enthält.

5. System nach Anspruch 1 bis 4, dadurch gekennzeichnet, daß der Anteil aus Peroxoverbindung, Alkylglucosid und gegebenenfalls damit verträglichem nichtionischen Tensid noch saure Hilfsstoffe enthält, die den pH-Wert während der Desinfektion und Reinigung auf 2 - 7, vorzugsweise 3,5 einstellen.

6. System nach Anspruch 1 bis 5, dadurch gekennzeichnet, daß das Verhältnis der mittleren Molmassen von Peroxoverbindung zu Alkylglucosid $1 . 10^2 : 1$ bis $32 . 10^4 : 1$, vorzugsweise $1 . 10^3 : 1$ bis $32 . 10^3 : 1$ und insbesondere $1 . 10^3 : 1$ bis $8,3 . 10^3 : 1$ beträgt.

7. System nach Anspruch 1 bis 6, dadurch gekennzeichnet, daß der $H_2O_2$-neutralisierende Anteil aus Reduktionsmitteln oder Katalysatoren, vorzugsweise aus Reduktionsmitteln und Katalysatoren besteht.

8. System nach Anspruch 1 bis 7, dadurch gekennzeichnet, daß der $H_2O_2$-neutralisierende Anteil mit wasserlöslichen filmbildenden Polymeren besprüht ist.

9. System nach Anspruch 1 bis 7, dadurch gekennzeichnet, daß der peroxoverbindungshaltige Anteil und/oder der $H_2O_2$-neutralisierende Anteil in gelöster Form, als Pulver oder als Tablette vorliegen.

10. Verfahren zur Reinigung von Kontaktlinsen unter Verwendung des Systems nach den Ansprüchen 1 bis 9.

**Claims**

1. A disinfecting and cleaning system for contact lenses containing solid peroxo compounds, reducing agents and/or catalysts and surfactants, characterized in that the peroxo-containing component contains an addition of alkyl glucoside and the H$_2$O$_2$-neutralizing component an addition of buffer salts to adjust a pH of 7.

2. A system as claimed in claim 1, characterized in that the peroxo-containing component consists of potassium persulfate or urea peroxohydrate.

3. A system as claimed in claims 1 and 2, characterized in that the alkyl glucoside corresponds to the general summation formula R,O (Z)$_x$ , where R$_1$ is a C$_8$-C$_{18}$ and prefer-ably C$_{12}$-C$_{14}$ alkyl chain, Z is glucose and x = 1 to 10 and preferably 1 to 5.

4. A system as claimed in claims 1 to 3, characterized in that the peroxo compound/alkyl glucoside component contains an addition of a nonionic surfactant compatible therewith.

5. A system as claimed in claims 1 to 4, characterized in that the component of peroxo compound, alkyl glucoside and, optionally, nonionic surfactant compatible therewith also contains acidic auxiliaries which establish a pH value of 2 - 7, preferably 3.5, during disinfection and cleaning.

6. A system as claimed in claims 1 to 5, characterized in that the ratio of the average molecular weights of peroxo compound to alkyl glucoside is from 1 ` 10$^2$ : 1 to 32 ` 10$^4$ : 1, preferably from 1 ` 10$^3$ : 1 to 32 ` 10$^3$ : 1 and more preferably from 1 ` 10$^3$ : 1 to 8.3 ` 10$^3$ : 1.

7. A system as claimed in claims 1 to 6, characterized in that the H$_2$O$_2$-neutralizing component consists of reducing agents or catalysts and preferably of reducing agents and catalysts.

8. A system as claimed in claims 1 to 7, characterized in that the H$_2$O$_2$-neutralizing component is sprayed with water-soluble film-forming polymers.

9. A system as claimed in claims 1 to 7, characterized in that the peroxo-containing component and/or the H$_2$O$_2$-neutralizing component are present in dissolved form, in powder form or in tablet form.

10. A process for cleaning contact lenses using the system claimed in claims 1 to 9.

**Revendications**

1. Système de désinfectants et de nettoyants pour lentilles de contact contenant des composés peroxo solides, des réducteurs et/ou des catalyseurs et des tensioactifs, caractérisé en ce que la composant contenant le composé peroxo comprend en supplément un alkylglucoside et la composante neutralisant 1 'eau oxygénée, des sels tampons pour ajuster le pH à 7.

2. Système selon la revendication 1, caractérisé en ce que la composante contenant le composé peroxo est constituée de persulfate de potassium ou de peroxohydrate d'urée.

3. Système selon les revendications 1 et 2, caractérisé en ce que l'alkylglucoside correspond à la formule brute générale R$_1$0 (Z)$_x$, dans laquelle R$_1$ représente une chaine alkyle comportant 8 à 18, de préférence 12 à 14 atomes de c, 2 correspond au glucose et x est égal à 1 à 10, de préférence à 1 à 5.

4. Système selon les revendications 1 à 3, caractérisé en ce que la composante de composé peroxo et d'alkylglucoside comporte en supplément un tensioactif non ionique compatible avec celle-ci.

5. Système selon les revendications 1 à 4, caractérisé en ce que la composante de composé peroxo et d'alkylglucoside et, éventuellement, d'un tensioactif compatible avec ceux-ci, contient encore en plus des adjuvants acides qui ajustent le pH pendant la désinfection et le nettoyage à 2 à 7, de préférence à 3.

6. Système selon les revendications 1 à 5, caractérisé en ce que le rapport des masses molaires du composé peroxo et de l'alkylglucoside atteint 1.10$^2$:1 à 32.10$^4$:1. de préférence 1.10$^3$:1 à 32.10$^3$:1 et, en particulier, 1.10$^3$:1 à 8,3.10$^3$:1.

7. Système selon les revendications 1 à 6, caractérisé en ce que la composante neutralisant l'eau oxygénée est constituée de réducteurs ou de catalyseurs, de préférence de réducteurs et de catalyseurs.

8. Système selon les revendications 1 à 7, caractérisé en ce que la composante neutralisant l'eau oxygénée est pulvérisée par des polymères filmogènes solubles dans l'eau.

9. Système selon les revendications 1 à 8, caractérisé en ce que la composante contenant le composé peroxo et/ou la composante neutralisant l'eau oxyénée est présente sous forme dissoute, en poudre ou en comprimé.

10. Procédé de nettoyage des lentilles de contact mettant en oeuvre le système selon les revendications 1 à 9.